(19) Europäisches Patentamt
European Patent Office
Office européen des brevets

(11) Veröffentlichungsnummer: **0 073 374**
**B1**

# (12) EUROPÄISCHE PATENTSCHRIFT

(45) Veröffentlichungstag der Patentschrift: **15.01.86**

(51) Int. Cl.⁴: **C 07 D 319/08**, C 08 L 15/00

(21) Anmeldenummer: **82107308.7**

(22) Anmeldetag: **12.08.82**

## (54) Verwendung cyclischer Acetale als nichtverfärbende Ozonschutzmittel.

(30) Priorität: **25.08.81 DE 3133567**

(43) Veröffentlichungstag der Anmeldung: **09.03.83 Patentblatt 83/10**

(45) Bekanntmachung des Hinweises auf die Patenterteilung: **15.01.86 Patentblatt 86/3**

(84) Benannte Vertragsstaaten: **CH DE GB LI**

(56) Entgegenhaltungen:
**CH-A-377 328**
**FR-A-1 387 099**
**US-A-3 784 594**

**CHEMICAL ABSTRACTS, Band 92, 1980, Seite 626, Nr. 75936t, Columbus, Ohio, USA. F. KASPER et al.: "Bicyclic compounds. Part XI. Reactions of 2,2-bis(hydroxymethyl) bicyclo(2.2.1)hept-5-ene"**

(73) Patentinhaber: **BAYER AG, Konzernverwaltung RP Patentabteilung, D-5090 Leverkusen 1 Bayerwerk (DE)**

(72) Erfinder: **Roos, Ernst, Dr., Am Geus Garten 6, D-5068 Odenthal- Osenau (DE)**
Erfinder: **Jeblick, Werner, Dr., Walter Flex-Strasse 30, D-5090 Leverkusen 1 (DE)**
Erfinder: **Ruetz, Lothar, Dr., Wilhelm Busch-Strasse 37, D-4047 Dormagen 5 (DE)**

**EP 0 073 374 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der Erfindung ist ein Verfahren zur Stabilisierung von natürlichem und/oder synthetischem Kautschuk gegen Ozonabbau durch Zusatz von Ozonschutzmitteln, das dadurch gekennzeichnet ist, daß man als Ozonschutzmittel ein Kondensationsprodukt aus einem Diol mit Aldehyden der Formel

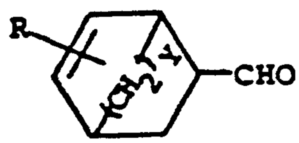

in denen R Wasserstoff oder Methyl bedeutet, wobei die Methylgruppe in 3- oder 4-Stellung stehen kann und Y den Zahlenwert O oder 1 annimmt, in wirksamen Mengen einsetzt.

Bevorzugte Diole entsprechen der Formel

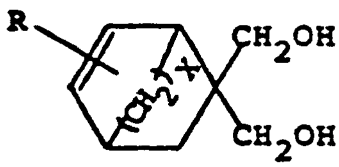

in der R Wasserstoff oder Methyl bedeutet, wobei die Methylgruppe in 3- und/oder 4-Stellung stehen kann und X den Zahlenwert O oder 1 annimmt.

Beispiele für die Aldehyde sind:

Tetrahydro-$\Delta^3$-benzaldehyd, 3-Methyltetrahydro- $\Delta^3$-benzaldehyd, 4-Methyltetrahydro-$\Delta^3$-benzaldehyd, 3,4-Dimethyltetrahydro-$\Delta^3$-benzaldehyd, 2,5-Endomethylen-tetrahydro-$\Delta^3$-benzaldehyd, 2,5-Endomethylen-3-methyl-tetrahydro-$\Delta^3$-benzaldehyd, 2,5-Endomethylen-4-methyl-tetrahydro-$\Delta^3$-benzaldehyd, 2,5-Endomethylen-3,4-dimethyltetrahydro-$\Delta^3$-benzaldehyd.

Die Kondensation der genannten Diole mit den Tetrahydro- $\Delta^3$-benzaldehyden kann in analoger Weise nach den für die Acetalisierung von Alkoholen mit Aldehyden bekannten Verfahren erfolgen.

Man setzt die Polyhydroxylverbindung mit dem Aldehyd bevorzugt in Gegenwart katalytischer Mengen eines sauren Dehydratisierungskatalysators bei Temperaturen von 0 - 200°C, vorzugsweise 20 - 120°C, um, wobei pro Mol Alkohol 1 Mol Aldehyd eingesetzt wird.

Die Reaktion kann ohne Lösungsmittel oder in Gegenwart von Lösungsmitteln durchgeführt werden. Als Lösungsmittel sind sowohl polare, wie Wasser, Methanol, Ethanol oder Dioxan, als auch unpolare, wie Benzin, Benzol oder Toluol, geeignet. Bei Verwendung von mit Wasser nicht mischbaren Lösungsmitteln können diese gegebenenfalls zur azeotropen Destillation des bei der Kondensation entstehenden Wassers benutzt werden. Als saure Dehydratisierungskatalysatoren seien HCl, $ZnCl_2$, $H_2SO_4$, Benzolsulfonsäure, Naphthalinsulfonsäure oder p-Toluolsulfonsäure, bevorzugt p-Toluolsulfonsäure erwähnt.

Die Katalysatoren werden bevorzugt in einer Dosierung von 0,05 bis 5, besonders bevorzugt von 0,1 bis 1 Gew.-%, bezogen auf den Aldehyd, eingesetzt.

Die Kondensationsprodukte lassen sich leicht in Kautschuk-Mischungen verteilen und können in Verbindung mit den üblicherweise benutzten Kautschuk-Chemikalien (z.B. Vulkanisationsbeschleunigern, Vulkanisiermitteln, Alterungsschutzmitteln, Weichmachern, Füllstoffen, Wachsen, Farbstoffen usw.) verwendet werden, ohne diese in ihrer spezifischen Wirkung zu beeinträchtigen.

Die Dosierung der Kondensationsprodukte in Polychloropren-Kautschuk liegt zwischen 0,1 bis 6,0 Gew.-%, vorzugsweise 0,3 bis 3,0 Gew.-%, bezogen auf den Polymergehalt, der aus 100,0 Gew.-% Polychloropren oder Polychloropren mit einem covulkanisierbaren Kautschuk besteht, wobei der Mindestgehalt an Polychloropren 20 Gew.-%, vorzugsweise 30 %, beträgt.

Geeignete, mit Polychloropren covulkanisierbare Kautschuke sind z.B. Naturkautschuk oder synthetische kautschukähnliche Polymere, die noch Doppelbindungen enthalten und die z.B. aus konjugierten Diolefinen, wie Butadien, Dimethylbutadien, Isopren und seinen Homologen erhalten werden oder Mischpolymerisate derartig konjugierter Diolefine mit polymerisierbaren Vinylverbindungen, wie z.B. Styrol, α-Methylstyrol, Acrylnitril, Methacrylnitril, Acrylaten, Methacrylaten.

Werden die Kondensationsprodukte anderen Kautschuktypen als den erwähnten Polychloroprenen zugesetzt, so empfiehlt sich eine Kombination mit Wachsen, da eine solche Kombination eine synergistische Wirkung zeigt.

Das Gewichtsverhältnis von Wachs zu den erfindungsgemäßen Kondensationsprodukten kann in weiten Grenzen schwanken, bevorzugt liegt es zwischen 0,25 und 2,5 zu 1.

Die Wachse bestehen mindestens teilweise aus mikrokristallinen Paraffinen. Makrokristalline Paraffine sind solche, deren Refraktionsindex $n_D^{100}$ niedriger liegt als der nach der Gleichung:

$n_D^{100} = 0{,}00035\,t + 1{,}4056$ (t = Erstarrungspunkt in °C) berechnete, während mikrokristalline Paraffine solche darstellen, deren Refraktionsindex höher liegt als der nach der obigen Formel berechnete (vergleiche auch für die Definition Petroleum Waxes, Proceedings of ASTM-TAPPI Symposium on Petroleum Waxes, Febr. 63, TAPPI-STAP No. 2, S. 1 bis 19).

Kombinationen aus den erfindungsgemäßen cyclischen Acetalen von Diolen und Wachsen stellen beispielsweise die folgenden dar: 4 Gew.-Teile eines der eingangs als bevorzugt aufgeführten Kondensationsprodukte + 2,0 Gew.-Teile mikrokristallines Paraffin;

Geeignete Kautschuke sind Naturkautschuk oder, außer Polychloropren synthetische, kautschukähnliche Polymere, die noch Doppelbindungen enthalten und die z.B. aus konjugierten Diolefinen; wie Butadien, Dimethylbutadien, Isopren und seinen Homologen, erhalten werden, oder Mischpolymerisate derartig konjugierter Diolefine mit polymerisierbaren Vinylverbindungen, wie Styrol, α -Methylstyrol, Acrylnitril, Methacrylnitril, Acrylaten und Methacrylaten.

Die synergistisch wirkende Ozonschutzwachskombination wird in solchen Mengen den Kautschuken zugesetzt, daß eine Stabilisierung gegen den Abbau durch Ozon eintritt. Die entsprechenden Mengen sind dem Durchschnittsfachmann geläufig oder leicht zu ermitteln. Die Dosierung liegt beispielsweise zwischen 0,5 bis 5 Gew.-%, vorzugsweise zwischen 1 bis 10 Gew.-%, bezogen auf den Polymergehalt.

**Beispiel 1**

Herstellung des Kondensationsproduktes:

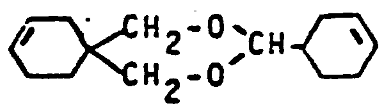

71 g (0,5 Mol) 1,1-Di-hydroxymethyl-$\Delta^3$-cyclo-hexen, 55 g (0,5 Mol) Tetrahydro- $\Delta^3$-benzaldehyd und 3 g p-Toluolsulfonsäure (p-Tssre) wurden in 500 ml Toluol am Wasserabscheider gekocht, bis kein Wasser mehr azeotrop abgeschieden wurde. Die Toluollösung wurde mit 5 g $NaHCO_3$ verrührt, um die p-Tssre zu neutralisieren. Das Gemisch wurde filtriert, die Lösung durch Ausdestillieren bei 80°C/20 mbar vom Lösungsmittel befreit. Das zurückbleibende Öl wurde im Hochvakuum destilliert.

Ausbeute 71,5 g = 61 % d.Th. farbloses Öl

Kp 0,2 mbar/105-110°C, $n_D^{20}$ 1 5109

**Beispiel 2**

Herstellung von:

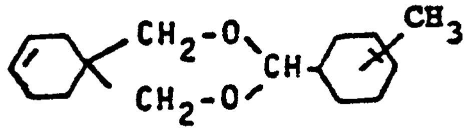

85,2 g (0,6 Mol) 1,1-Di-hydroxymethyl-$\Delta^3$-cyclohexen, 78 g (0,6 Mol) 3- und 4-Methyl-tetrahydro-$\Delta^3$-benzaldehyd und 3 g p-Tssre wurden in 300 ml Methanol 8 Stunden unter Rückfluß gekocht. Das Gemisch wurde mit einer Lösung von 6 g $NaHCO_3$ in 300 ml Wasser verrührt, wobei sich ein Öl ausschied. Dieses wurde abgetrennt und im Hochvakuum destilliert.

Ausbeute: 107 g = 72 % d.Th. farbloses Öl

Kp 0,2 mbar/120-125°C, $n_D^{20}$ 1.5098

**Beispiel 3**

Herstellung von:

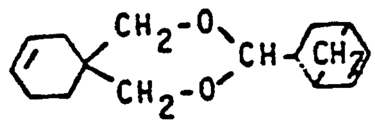

71 g (0,5 Mol) 1,1-Di-hydroxymethyl-Δ3-cyclohexen, 61 g (0,5 Mol) 2,5-Endomethylen-tetrahydro-Δ3-benzaldehyd und 1 g p-Tssre wurden in 500 ml Cyclohexan am Wasserabscheider gekocht, bis kein Wasser mehr azeotrop überging. Das Gemisch wurde mit 2 g $NaHCO_3$ neutralisiert, filtriert und bei 80° C/20 mbar ausdestilliert. Das zurückbleibende Öl wurde im Hochvakuum destilliert.

Ausbeute: 77,6 % d.Th. farbloses Öl Kp 0,09 mbar/ 121° C, welches zu farblosen Kristallen Fp. 36-38° C erstarrte.

**Beispiel 4**

Herstellung von:

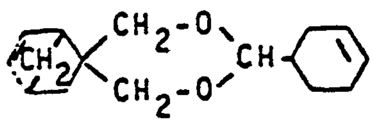

46,2 g (0,3 Mol (1,1-Di-hydroxymethyl-2,5-endomethylen-Δ3-cyclohexen 34,8 g (0,3 Mol) Tetrahydro-Δ 3-benzaldehyd und 1 g p-Tssre wurden in 200 ml Wasser 5 Stunden unter Rückfluß gekocht. Das ausgeschiedene Öl wurde in Methylenchlorid aufgenommen und mit einer Lösung von 2 g $NaHCO_3$ in 200 ml Wasser ausgeschüttelt. Nach Abtrennen der Methylenchloridlösung und Entfernen des Lösungsmittels blieb ein Öl zurück, welches im Hochvakuum destilliert wurde.

Ausbeute: 66 g = 89 % d.Th. farbloses Öl

Kp 0,2 mbar/110° C, $n_D^{20}$ 1.5170

**Beispiel 5**

Herstellung von:

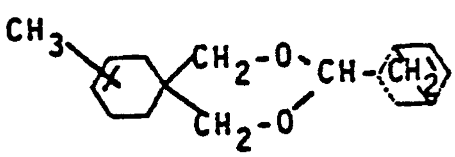

93,6 g (0,6 Mol) 1,1-Di-hydroxymethyl-3- und 4-Methyl-Δ3-cyclohexen, 73,2 g (0,6 Mol) 2,5-Endomethylentetrahydro- Δ3-benzaldehyd und 3 g P-Tssre wurden in 300 ml Toluol 4 Stunden am Wasserabscheider gekocht. Zur Neutralisation wurde das Gemisch mit 6 g $Ca(OH)_2$ verrührt und filtriert. Nach Ausdestillieren der Toluollösung bleibt ein Öl zurück, welches im Hochvakuum destilliert wurde.

Ausbeute 124 g = 81,3 % d.Th. farbloses Öl

Kp 0,1 mbar/121° C, $n_D^{20}$ 1.5135

**Beispiel 6**

Herstellung von:

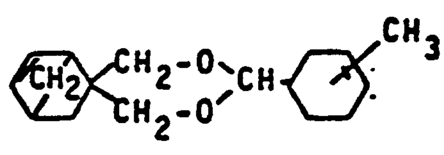

92,4 g (0,6 Mol) 1,1-Di-hydroxymethyl-2,5-endomethylen-$\Delta^3$-cyclohexen, 78 g (0,6 Mol) 3- und 4-Methyltetrahydro-$\Delta^3$-benzaldehyd und 3 g p-Tssre wurden in 300 ml Methanol 8 Stunden unter Rückfluß gekocht. Neutralisation mit 6 g $NaHCO_3$ in 300 ml Wasser wurde das ausgeschiedene Öl abgetrennt und im Hochvakuum destilliert.

Ausbeute: 127 g = 81,4 % d.Th. farbloses Öl

Kp 0,17 mbar/120-125°C

**Beispiel 7**

Herstellung von:

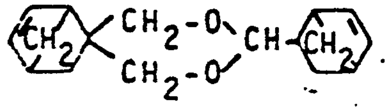

77 g (0,5 Mol) 1,1-Di-hydroxymethyl-2,4-endomethylen-$\Delta^3$-vyclohexen, 77 g (0,5 Mol) 2,4-Endomethylentetrahydro-$\Delta^3$-benzaldehyd und 2 g p-Tssre wurden in 300 ml Wasser 5 Stunden unter Rückfluß gekocht. Beim Erkalten schieden sich fast farblose Kristalle aus. Diese wurden abgesaugt und getrocknet.

Ausbeute: 120 g = 93 % d.Th. leicht beige Kristalle Fp 161-162°C.

**Beispiel A**

Folgende Kautschukmischung wurde auf der Walze hergestellt:

Polychloropren 100,0 Gew.-Teile
Magnesiumoxid 4,0 Gew.-Teile
Stearinsäure 0,5 Gew.-Teile
Gefällte Kieselsäure (BET-Oberfläche 180 $m^2/g$) 20,0 Gew.-Teile
Weichkaolin 170,0 Gew.-Teile
Titandioxid 5,0 Gew.-Teile
Antimonoxid 5,0 Gew.-Teile
Naphthenischer Mineralölweichmacher 20,0 Gew.-Teile
Chlorparaffin 10,0 Gew.-Teile
Ethylenthioharnstoff 1,2 Gew.-Teile
Zinkoxid 5,0 Gew.-Teile
Ozonschutzmittel 0,5 Gew.-Teile

Von diesen Mischungen wurden Prüfkörper von 0,4 x 4,5 x 4,5 cm und 0,4 x 4,5 x 5,5 cm vulkanisiert (Preßvulkanisation 30 min. bei 150°C). Je 4 Prüfkörper wurden dann in einen Kunststoffrahmen so eingespannt, daß an der Oberfläche Dehnungen von 10, 20, 30 und 60 % entstanden. Die gespannten Prüfkörper wurden mit einem Luftstrom, der 1000 Teile Ozon auf 100 Millionen Teile Luft enthielt, bei Raumtemperatur behandelt. Nach jeweils 2, 4, 6, 8, 24, 48, 72, 86 und 168 Stunden wurden die Proben mit dem Auge auf eventuelle Risse untersucht. In den nachfolgenden Tabellen sind jeweils die Stunden bis zur ersten Rißbildung eingetragen. Nach 168 Stunden wurden die Versuche abgebrochen.

**Tabelle 1**

| Produkt nach Beispiel | Dehnung in % | | | |
|---|---|---|---|---|
| | 10 | 20 | 30 | 60 |
| ohne Ozonschutzmittel (Vergleich) | 48 | 24 | 8 | 6 |
| 1 | >168 | >168 | >168 | >168 |
| 2 | >168 | >168 | >168 | 24 |
| 3 | >168 | >168 | >168 | >168 |
| 4 | >168 | >168 | >168 | >168 |
| 5 | >168 | >168 | >168 | >168 |
| 6 | >168 | >168 | >168 | 24 |
| 7 | >168 | >168 | >168 | >168 |

**Beispiel B**

Folgende Kautschukmischung wurde auf der Walze hergestellt:
Naturkautschuk 100,0 Gew.-Teile
Zinkoxid 10,0 Gew.-Teile
Gefällte Kreide 160,0 Gew.-Teile
Titandioxid 10,0 Gew.-Teile
Stearinsäure 0,7 Gew.-Teile
Ozonschutzwachs 2,0 Gew.-Teile
Dibenzothiazyldisulfid 1,0 Gew.-Teile
Hexamethylentetramin 0,25 Gew.-Teile
Schwefel 2,2 Gew.-Teile
Ozonschutzmittel 4,0 Gew.-Teile

Die Prüfkörper wurden in der presse 30 min. bei 140° C vulkanisiert. Die prüfung wurde ebenso durchgeführt wie im Beispiel A beschrieben, allerdings betrug die Ozonkonzentration statt 1000 Teile jetzt 100 Teile pro 100 Millionen Teile Luft.

**Tabelle 2**

| Produkt nach Beispiel | Dehnung in % | | | |
|---|---|---|---|---|
| | 10 | 20 | 30 | 60 |
| ohne Ozonschutzmittel (Vergleich) | 2 | 2 | 2 | 2 |
| 1 | 48 | 48 | 8 | 8 |
| 2 | > 168 | > 168 | 4 | 2 |
| 3 | 48 | 24 | 8 | 8 |
| 4 | > 168 | 48 | 48 | 8 |
| 6 | > 168 | > 168 | 4 | 4 |
| 7 | > 168 | > 168 | 48 | 24 |

**Beispiel C**

Folgende Kautschukmischung wurde auf der Walze hergestellt:
Styrol-Butadien-Mischpolymerisat 100,0 Gew.-Teile
Zinkoxid 5,0 Gew.-Teile
Ruß (N 220) 55,0 Gew.-Teile
Naphthenischer Mineralölweichmacher 2,0 Gew.-Teile
Hocharomatischer Mineralölweichmacher 2,0 Gew.-Teile
Stearinsäure 2,0 Gew.-Teile
Ozonschutzwachs 2,0 Gew.-Teile
Benzothiazyl-2-cyclohexylsulfenamid 1,3 Gew.-Teile
Schwefel 1,6 Gew.-Teile
Ozonschutzmittel 4,0 Gew.-Teile

Die Prüfkörper wurden in der Presse 30 min. bei 150° C vulkanisiert. Die Prüfung wurde ebenso durchgeführt wie in Beispiel A beschrieben, allerdings betrug die Ozonkonzentration statt 1000 Teile jetzt 100 Teile pro 100 Millionen Teile Luft.

**Tabelle 3**

| Produkt nach Beispiel | Dehnung in % | | | |
|---|---|---|---|---|
| | 10 | 20 | 30 | 60 |
| ohne Ozonschutzmittel (Vergleich) | 4 | 2 | 2 | 2 |
| 1 | > 168 | >168 | 4 | 2 |
| 3 | > 168 | 6 | 4 | 2 |
| 4 | > 168 | > 168 | 6 | 2 |
| 7 | > 168 | > 168 | >168 | 2 |

**Beispiel D**

Folgende Kautschukmischung wurde auf der Walze hergestellt:

Styrol-Butadien-Mischpolymerisat 50,0 Gew.-Teile
Naturkautschuk 50,0 Gew.-Teile
Zinkoxid 5,0 Gew.-Teile
Gefällte Kreide 120,0 Gew.-Teile
Gefällte Kieselsäure (BET-Oberfläche: 180 $m^2/g$) 35,0 Gew.-Teile
Diethylenglykol 2,5 Gew.-Teile
Naphthenischer Mineralölweichmacher 10,0 Gew.-Teile
Stearinsäure 1,0 Gew.-Teile
Ozonschutzwachs 2,0 Gew.-Teile
Benzothiazyl-2-cyclohexylsulfenamid 0,8 Gew.-Teile
Diphenylguanidin 0,3 Gew.-Teile
Tetramethylthiuramdisulfid 0,3 Gew.-Teile
Schwefel 2,0 Gew.-Teile
Ozonschutzmittel 4,0 Gew.-Teile

Die Prüfkörper wurden in der Presse 15 min. bei 140°C vulkanisiert. Die Prüfung wurde ebenso durchgeführt wie in Beispiel A beschrieben, allerdings betrug die Ozonkonzentration statt 1000 Teile letzt 200 Teile pro 100 Millionen Teile Luft.

**Tabelle 4**

| Produkt nach Beispiel | Dehnung in % | | | |
|---|---|---|---|---|
| | 10 | 20 | 30 | 60 |
| ohne Ozonschutzmittel (Vergleich) | 6 | 6 | 6 | 6 |
| 1 | 72 | 72 | 72 | 24 |
| 3 | >168 | 48 | 24 | 8 |
| 4 | > 168 | 72 | 24 | 8 |
| 7 | > 168 | 72 | 24 | 8 |

**Patentansprüche**

1. Verfahren zur Stabilisierung von natürlichen und/ oder synthetischen Kautschuken gegen Ozonabbau durch Zusatz von Ozonschutzmitteln, dadurch gekennzeichnet, daß man als Ozonschutzmittel ein Kondensationsprodukt aus einem Diol mit Aldehyden der Formel

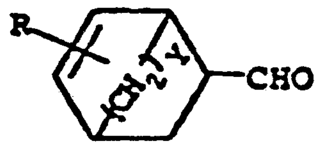

in der R Wasserstoff oder Methyl bedeutet, wobei die Methylgruppe in 3- und/oder 4-Stellung steht und Y den Zahlenwert O oder 1 annimmt, in wirksamen Mengen einsetzt.

2. Verfahren nach Anspruch 1, dadurch gekennzeichnet, daß das Diol der Formel

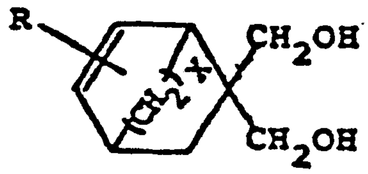

in der R Wasserstoff oder Methyl bedeutet, wobei die Methylgruppe in 3- und/oder 4-Stellung steht und X den Zahlenwert O oder 1 bedeutet, entspricht.

3. Verfahren nach Ansprüchen 1 und 2, dadurch gekennzeichnet, daß man das Kondensationsprodukt in Kombination mit Wachsen einsetzt.

**Claims**

1. Process for stabilising natural and/or synthetic rubbers against degradation under the effect of ozone by the addition of antiozonants, characterised in that a condensation product of a diol with aldehydes of the formula

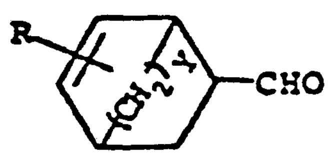

in which
R denotes hydrogen or methyl,
the methyl group being in the 3- and/or 4-position, and
Y has the numerical value 0 or 1, is used in effective quantities as the antiozonant.

2. Process according to Claim 1, characterised in that the diol corresponds to the formula

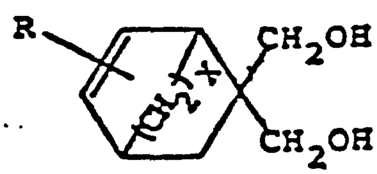

in which
R denotes hydrogen or methyl,
the methyl group being in the 3- and/or 4-position,
and X denotes the numerical value 0 or 1.

3. Process according to Claims 1 and 2, characterised in that the condensation product is used in combination with waxes.

**Revendications**

1. Procédé de stabilisation de caoutchoucs naturels et/ou synthétiques contre la dégradation par l'ozone, par addition d'agents anti-ozone, caractérisé en ce qu'on utilise en quantités efficaces comme agent anti-ozone un produit de condensation d'un diol avec des aldéhydes de formule

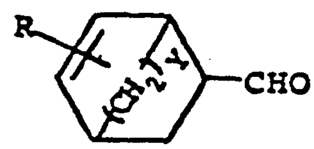

dans laquelle R désigne l'hydrogène ou le groupe méthyle, le groupe méthyle étant en position 3 et/ou 4 et Y prenant la valeur 0 ou 1.

2. Procédé suivant la revendication 1, caractérisé en ce que le diol répond à la formule

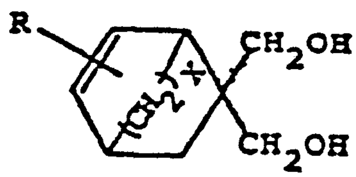

dans laquelle R désigne l'hydrogène ou le groupe méthyle, le groupe méthyle étant en position 3 et/ou 4 et X ayant la valeur 0 ou 1.

3. Procédé suivant les revendications 1 et 2, caractérisé en ce qu'on utilise le produit de condensation en association avec des cires.